Europäisches Patentamt

European Patent Office

Office européen des brevets

(11) Publication number: **0 566 205 A1**

# EUROPEAN PATENT APPLICATION

(21) Application number: **93201079.6**

(22) Date of filing: **14.04.93**

(51) Int. Cl.5: **C07K 15/00, G01N 33/531, G01N 33/543, G01N 33/546**

(30) Priority: **17.04.92 JP 98239/92**

(43) Date of publication of application:
**20.10.93 Bulletin 93/42**

(84) Designated Contracting States:
**AT BE CH DE DK ES FR GB GR IE IT LI LU MC NL PT SE**

(71) Applicant: **AKZO N.V.**
**Velperweg 76**
**NL-6824 BM Arnhem(NL)**

(72) Inventor: **Iwata, Keisuke**
**1192-2 Ooge Kuki-shi**
**Saitama 346(JP)**
Inventor: **Aoki, Yoko**
**408 Asahipuraza-Araikekoen,**
**601 Atago-machi**
**Koriyama-shi Fukushima 963(JP)**

(74) Representative: **Hermans, Franciscus G.M. et al**
**P.O. Box 20**
**NL-5340 BH Oss (NL)**

(54) Method for the elimination of non-specific reactions in immuno-assays.

(57) An antibody for immunoassay, of which Fc portion is blocked by a blocking agent comprising at least an antigen-binding site of an immunoglobulin specifically bindable to the Fc portion, a reagent for immunoassay comprising said antibody, an immunoassay using said immunoassay reagent and a blocking agent for blocking Fc portion. It is possible to eliminate non-specific reactions by substances such as RF in immunoassay.

EP 0 566 205 A1

DETAILED DESCRIPTION OF THE INVENTION

The present invention relates to an antibody for eliminating a pseudoreaction that is caused by a rheumatoid factor (to be referred to as RF hereinafter) binding to the Fc portions of antibodies in immunoassay. Particularly, the present invention relates to an antibody for immunoassay which is blocked at its Fc portion which may cause a non-specific response leading to a pseudoreaction, to a reagent for immunoassay that comprises said antibodies, to an immunoassay using said reagent, and to a blocking agent for blocking Fc portion for use in eliminating the non-specific reaction.

An immunoassay, i.e., an in vitro assay based on the usage of the binding response between antigens and antibodies makes a specific detection of minute contents or precise determination of minute contents practicable so that it is widely used in laboratory tests. An immunoassay, dependent on the labeling method employed, can be an SRID, agglutination (based on the usage of carriers such as blood cells or latex), radioimmunoassay (RIA), enzymeimmunoassay (EIA), fluoroimmunoassay (FIA), nephlometory, turbidimetrical immunoassay, etc.

However, it is a well-known problem that in the application of those immunoassays as laboratory tests, RF or Clq complement that exists in a biological fluid from a patient such as blood, urine and the like can cause non-specific agglutination reaction, which may result in a pseudopositive reaction.

Hence, to eliminate such non-specific reaction due to RF and others, modifications of immunoassay have been proposed wherein the sample is pretreated with dithiothreitol or 2-mercaptoethanol or wherein the sample is pretreated by addition of $\gamma$-globulin or its degenerate product, or wherein a fragment of an antibody such as Fab, F(ab'), or the like is used.

For example, in Journal of Clinical Microbiology, Feb. 1976, p. 157-160, a method was described wherein the sample is pretreated with dithiothreitol. Japanese Patent Publication No. 61-942 (1986) discloses a method wherein $\gamma$-globulin or its degenerate product from an unsensitized normal animal is added to the sample, and an antigen or antibody in the sample is immunologically determined in the presence of such immunoactive substance.

As regards the use of the fragment of the antibody, for example, Japanese Patent Publication No. 63-38668 (1988) discloses an immunoassay which uses, as the antibody for immunoassay, a F(ab')$_2$ fragment of an immunoglobulin where the Fc portion is cleaved off, because the Fc portion is involved in the non-specific reaction.

Particularly, for an agglutination based on a usage of latex, such methods are known in the art where non-specific reactions due to RF and other substances are prevented by adding a derivative of urea, formamide, etc., and lower alkylsulfoxide and the like to the sample (Japanese Patent Publication No. 60-4941 (1985)), or where the interference by RF and the like is avoided by using latex particles that are sensitized with a mixture comprising F(ab')$_2$ and Fc fragments, or Facb and pFc' fragments of immunoglobulins (Japanese Patent Publication No. 63-63863 (1988) and Japanese Patent Publication No. 63-41426 (1988)).

However, the modification comprising the pretreatment has drawbacks associated with increased steps for measurement and reduced sensitivity. The immunereactivity of an antibody fragment such as Fab, F-(ab')$_2$, etc. that has been obtained by hydrolytically treating the antibody, will be adversely effected by such a treatment.

The present inventors have strenuously studied for finding a solution to above problems, and found a method for eliminating the non-specific reaction due to the interference by RF in immunoassay, based on a novel principle.

According to the present invention, it is possible to eliminate non-specific reactions due to substances such as RF. Hence, it is possible to achieve elimination of pseudopositivity and improvement of specificity, and thus to generate more reliable laboratory tests.

Further, according to the present invention, no pretreatment of the sample itself is necessary, thus enabling quick completion of laboratory tests, and eliminating the drawbacks resulting from reduced sensitivity. Moreover, because it is unnecessary to subject the antibody, to enzymatic hydrolysis in order to avoid the influence from substances such as RF, the adverse effects (i.e., reduced sensitivity and reduced stability of the antibody) of the enzymatic hydrolysis on the immunoassay antibody are avoided.

The present invention provides an antibody for immunoassay, of which the Fc portion is blocked by a blocking agent comprising at least one antigen-binding site of an immunoglobulin specifically bindable to the Fc portion. That is, the present invention provides an antibody of which Fc portion, is blocked based on antigen-antibody reaction and which does not unspecifically react with substances such as RF.

Further, the present invention provides a reagent for immunoassay comprising the above antibody.

2

Furthermore, the present invention provides an immunoassay using the above reagent. According to the immunoassay of the present invention, the non-specific reaction of the antibody with such substance as RF can be eliminated.

Besides, the present invention provides a blocking agent for blocking Fc portion of an antibody for immunoassay, thereby eliminating interference by substances such as RF and others, the blocking agent comprising at least one antigen-binding site of an immunoglobulin specifically bindable to the Fc portions.

In the present invention, the immunoassay is an assay based on the usage of immunologic reactions (antigen-antibody reaction), and includes both quantitative and qualitative determination. Examples of immunoassay include single radial immunodiffusion (SRID), agglutination (agglutination based on the usage of carriers such as latex, blood cells, etc.), radioimmunoassay (RIA), enzymeimmunoassay (EIA), turbidimetrical immunoassay (TIA), immunonephlometry, fluoroimmunoassay, luminesceimmunoassay, and turbidimetry, nephlometry and particle size distribution measurment based on agglutination technique.

The antibody according to the invention, of which Fc portion is blocked, is an antibody that is bindable to a test substance in the sample used in these immuno-assays. For example, antibodies derived from mouse, rabbit, guinea pig, sheep, goat and others, and antibodies of IgG, IgM, etc. classes can be used.

Because RFs are ready to bind to the Fc portions of antibodies, the authentic antigen-antibody reaction are interfered by the presence of RFs, thus leading to wrong measurements of the substance of interest in a biological fluid. This, especially in the agglutination test, may often give pseudopositivity that results from non-specific agglutination.

The present invention gives a solution to this problem, by blocking in advance the Fc portion of the antibody to which RF binds specifically.

The blocking agent of the present invention specifically blocks the Fc portion of an antibody for immunoassay, thereby eliminating the interference by RF, and comprises at least one antigen-binding site of an immunoglobulin, capable of binding specifically to the Fc portion of the antibody. The antigen-binding site corresponds to variable region ($V_L$, $V_H$ regions) that can recognize an antigen and bind thereto.

As an example of such blocking agent, the whole body of an immunoglobulin or its fragments can be mentioned. The examples of such fragments include Fv, Fab, Fab', $F(ab')_2$, Facb, and Fab/c.

Because said immunoglobulin or its fragment is used for eliminating the interference by RF, it is desirable that it has no site bindable to RF in itself. For example, Fv, Fab, Fab' or $F(ab')_2$ fragment can be used. Preferably, a low-molecular fragment such as Fab, Fab' or Fv is used. Further, in the agglutination, to minimize auxiliary actions of the blocking agent, preferred fragments are the ones having one antigen-binding site per fragment such as fragments where S-S bonds at the hinge region have been cleaved. Particularly, the fragments such as Fab or Fab' are representative. Furthermore, these fragments may be modified with other compounds.

There are no special limitations as to the origin and class of the immunoglobulin that can be used as blocking agent and immunoglobulins from mouse, rabbit, rat, guinea pig, sheep, goat and chicken can be used, and those of IgG, IgM, etc. classes may be used. In addition, chimera immunoglobulins may also be used.

When IgG from chicken that does not react with RF is used as a blocking agent, consideration of the interference between the blocking agent and RF is not needed, so that its whole body or its desired fragment (for example, Fv, Fab, Fab', $F(ab')_2$, Facb, Fab/c) may be used.

Such immunoglobulins may be purchased as commercial products, or obtained from animals by using Fc fragment as an antigen for immunization of said animals. For example, such commercial products include goat anti-mouse IgG Fc fragment ($\gamma$ chain specific) (Organon Teknika KK), goat anti-rabbit IgG Fc fragment ($\gamma$ chain specific) (Organon Teknika KK), etc., and it may be chosen according to the antibody to be targeted. The procedures necessary for obtaining the immunoglobulin that has a desired specificity from animals is well known to those skilled in the art. As the Fc fragment of the antibody which is used as an antigen, given to the animal for preparing the blocking agent, any Fc fragments are adequate as far as they are derived from the same species of animal from which the antibody for immunoassay is derived. For the obtainment of such fragment it is not necessary to use the antibody to be used in the immunoassay as an antigen.

The fragment of the immunoglobulin may be obtained by digesting the immunoglobulin with a protease. The procedures for preparing the fragments of the immunoglobulin are well known to those skilled in the art, and described in, for example, "Continued series of biochemistry experiment 5: Methods for immunobiochemistry", Tokyo Chemistry Associates, 1986, pp. 89-99, and R.R. Porter, Biochem. J., 73, 119 (1959).

The proteases that may be used include papain, pepsin, plasmin, tripsin, etc. For example, digestion by papain may provide Fab fragments and digestion by pepsin may provide $F(ab')_2$ fragments.

The procedures for cleaving S-S bonds at the hinge region of the immunoglobulin or its fragments are known to those skilled in the art. For example, for cleaving the S-S bonds, such immunoglobulin or its fragments is treated with a reducing agent such as dithiothreitol, 2-mercaptoethanol or the like, and then with an SH reagent such as iodoaceto amide.

It is not always necessary to purify the thus obtained fragments, but it is preferred to separate and purify the fragments by ion exchange chromatography, gel filtration chromatography or the like.

The antibody for an immunoassay according to the present invention, that has been blocked, can be obtained by binding the blocking agent to a conventional antibody for immunoassay, thereby blocking the Fc portion of the antibody with the blocking agent.

The antibody for immunoassay of the present invention can be provided with an average of 0.5 to 6 molecules of blocking agent per molecule of the antibody. Preferably, the number of the blocking agents on an average is about 3 per molecule of the antibody.

For example, when an antibody used in the latex agglutination and adsorbed to latex is provided with a blocking agent comprising Fab or Fab' fragment preferably the average number of the Fab or Fab' fragments is 1.0 to 3.5 per molecule of the antibody. Further, when an antibody solution for EIA and a blocking agent comprising Fab or Fab' fragment are used, the average number of Fab or Fab' fragments per molecule of the antibody may be 5.0 to 6.0.

The binding of the antibody for immunoassay and the blocking agent can be achieved by combining both in a solution. The temperature for combination is usually 2 to 50 °C, preferably 4 to 37 °C. The time for combination is usually one minute to 2 days. The conditions appropriate for combination including ratio, temperature, time, etc. vary according to the affinity, antibody titer and purity of the immunoglobulin that is capable of binding specifically to the Fc portion of the antibody, and the concentration or density of the immunoassay antibody. For example, it is well known to those skilled in the art that the titer of the immunoglobulin material varies from lot to lot, and those skilled in the art can easily determine the appropriate temperature for combination and the time for combination, and modify the ratio for combination.

The reagent of the present invention comprises said antibody of which Fc portion is blocked.

As examples of reagents for an immunoassay, there are those that contain antibodies adsorbed to a carrier or to a substrate. As examples of the carrier, latex, blood cells, gold colloid, gelatin particles, etc. may be mentioned. As examples of the substrate, glass, plastics, membranes, etc. may be mentioned. Further, some reagents may comprise antibodies labeled with, for example, enzymes. Particularly, latex reagents, antibodies adsorbed to microplate, antibodies labeled with enzyme, etc. may be mentioned.

The reagent of the present invention may be obtained by combining a conventional immunoassay reagent comprising antibodies, with the blocking agent, and by providing the antibody with the blocking agent as described above.

The reagent of this invention may be treated by, for example, adding thereto a stabilizer such as bovine serum albumin.

The immunoassay of the present invention is an assay using the reagent of the present invention as described above.

The present immunoassay may be carried out by the same steps as in the assay using the conventional immunoassay reagent.

For instance, the latex agglutination test may be carried out by the following steps:

(1) One drop (e.g., 20 µl) of a sample, such as serum is placed within a circle of a test slide plate;

(2) One drop of a latex reagent comprising antibodies, of which Fc portions are blocked, is placed within the circle of the test slide plate;

(3) The test sample and the latex reagent are mixed with a stirring rod, and the test slide plate is gently rolled and

(4) After rolling for several minutes (e.g., 1 to 5 min.), it is checked visually whether agglutination is present or not, and, if present, the sample is regarded as positive.

Further, as an example of EIA, a microplate sandwich method can be carried out by the following steps:

(1) Calibrators and a sample are placed onto each well of an antibody-solid phase microplate that comprises antibodies of which Fc portions are blocked, and is incubated (e.g., at 37 °C for 15 minutes);

(2) The solution is aspirated from each well and washing solution is added thereto;

(3) The step 2 is repeated several times (e.g., 4 times) to wash the wells;

(4) Moisture is removed and enzyme-labeled antibodies, of which Fc portions are blocked, are added to each well, and incubated (e.g., at 37 °C for 15 min.);

(5) Step 2 is repeated several times (e.g., 4 times) to wash the wells;

(6) An reagent for enzyme reaction is added to each well, and incubated (e.g., at 37 °C for 10 min.);

(7) A stopper of enzyme reaction is added to stop the reaction;

(8) Absorbance of each well (e.g., at 450 nm) is determined using a reagent blank well as a reference;

(9) A calibration curve is prepared from measurements of the calibrators, and the content of the substance in the sample is determined.

In this case, if the Fc portions of either of the antibodies contained in the antibody-solid phase microplate in the step 1 or the antibodies in the enzyme-labeled antibody solution in the step 4 are blocked, satisfactory results are obtained. If the blocking is limited to only one of the two, it is preferred to block Fc portions of the antibodies of the antibody-solid phase microplate.

As can be understood from the above, it is possible to eliminate the interference by RF by replacing a conventional immunoassay reagent with the immunoassay reagent of the present invention, and where the immunoassay of the present invention is carried out by the same steps as a conventional immunoassay.

In the immunoassay of the present invention where blocked antibodies are used, a variety of samples can be tested. This method can be applied to any sample that can be studied by conventional immunoassay. As examples of such samples, those from humans such as human blood, urine, feces, etc. may be mentioned. Particularly, the method may be advantageously applied to a sample containing a higher amount of RF, like blood.

The analyte to be detected by the present immunoassay is not specially limited, and any kind of substance subjectable to a conventional immunoassay can be detected by the present immunoassay. As examples of such substances:

Tumor markers such as CEA, AFP, CA19-9, hCG, $\beta$2m, ferritin, etc.;

Coagulation fibrinolytic markers such as protein C, protein S, ATIII, FDP, D-dimer, etc.;

Infection marker such as CRP, ASO, HBs antigen, etc.;

Hormones such as TSH, prolactin, insulin, etc.;

Immunoglobulins and complements such as IgE, IgA, IgG, C3, C4, etc.; and

Tissue components such as myoglobin, myosin, etc. may be mentioned.

The present invention may likewise be applied in those cases where substances other than RF, (Fc binding substances) that can bind to the Fc portion of the immunoassay antibody and interfere with the immunologic reaction are present, and bring about the non-specific reaction in an assay. As examples of such Fc binding substances, complement Clq, Fc receptors, etc. may be mentioned.

The present invention will be illustrated by referring to the following Examples, but the present invention is not limited to the examples.

Example 1

Preparation of Fab blocking agent by papain digestion of goat IgG-anti-mouse IgG (Fc) antibody

Into 10 mM cystine-2 mM EDTA-0.1 M sodium phosphate buffer (pH 7.0) were dissolved 50 mg of goat IgG-anti-mouse IgG (Fc) antibody (immunoglobulin) and 0.5 mg of papain, and digestion was allowed to proceed at 37 °C for 75 minutes. Then, to the solution was added 6.3 mg of iodoaceto amide in a dark place, and the mixture was incubated at 37 °C for 15 min. The reaction mixture was dialyzed against 0.01 M sodium phosphate buffer (pH 8.0), the internal solution was collected, and the Fab fragments (blocking agents) were separated and purified therefrom using a DEAE-Sepharose column.

Example 2

Preparation of Fab' blocking agent by pepsin digestion and S-S bond cleavage of goat IgG-anti-rabbit IgG (Fc) antibody

In 0.1 M sodium acetate buffer (pH 4.0) were dissolved 50 mg of goat IgG-anti-rabbit IgG (Fc) antibody (immunoglobulin) and 1.7 mg of pepsin, digestion was allowed to proceed at 37 °C for 18 hours, and the solution was dialyzed against 10 mM tris-HC1 buffer (pH 8.0). 2-Mercaptoethanol was added to the internal solution to 10 mM, and the mixture was incubated at 37 °C for 75 min. Then iodoaceto amide was added to 10 mM in a dark place, and the reaction was allowed to proceed at 4 °C for 40 min. The reaction mixture was dialyzed against 50 mM glycine buffer (pH 8.6), and the internal solution was collected and the Fab' fragments (blocking agents) were separated and purified by a gel filtration.

Example 3

(1) Preparation of anti-D-Dimer antibody-sensitized latex reagent

In 9.5 ml of phosphate buffer was dissolved 0.75 mg of mouse IgG-anti-D-Dimer antibody, and 0.5 ml of polystylene latex (suspension containing 10 weight % solids) was added, and the mixture was stirred at room temperature for 2 hours. The sensitized latex was separated by centrifugation, and the supernatant was removed. The amount of the adsorbed IgG antibodies was determined by the following steps.

(a) The supernatant was filtrated with a filter of 0.1 μm, to remove a trace amount of latex possibly mixing therein.

(b) The concentration of unadsorbed IgG antibodies remaining in the filtrate was determined by measuring an absorbance at 280 nm.

(c) The unadsorbed amount determined at (b) was substracted from the amount of IgG antibodies used, and the result was regarded as the amount of IgG antibodies adsorbed to latex.

The precipitate was suspended in 10 ml of glycine buffer containing 2.25 mg of Fab fragment obtained according to the procedure as described in Example 1, to block the Fc portions of the mouse IgG antibodies. Further, the suspension was centrifuged, and the supernatant was removed. In the same manner as described above, the amount of bound Fab fragments was estimated.

The precipitate was suspended in bovine serum albumin solution (0.1 M glycine, 0.05 M sodium chloride, 0.05% sodium azide, and 0.2% BSA), to produce the anti-D-Dimer antibody-sensitized latex reagent.

Further, from the amount of IgG antibodies adsorbed to latex and the amount of Fab fragments bound to those IgG antibodies estimated as described above, the molecular ratio was calculated, and the result was regarded as the number (average) of the bound Fab fragments of the anti mouse IgG (Fc) antibodies per one mouse IgG antibody molecule.

(2) Non-specific reaction with RF

One drop of an RF positive human serum (653 IU/ml; International Immunology Corporation) and one drop of the anti-D-Dimer antibody-sensitized latex reagent prepared as described above were mixed, and the reaction plate was rolled gently, to observe the agglutination image, for 10 min. The results obtained from the above described procedure were compared to results obtained with anti-D-Dimer antibody-sensitized latex reagents differing in the number of the Fab fragments used (Example 1) to block the Fc portion of the mouse IgG antibody, as listed in Table 1.

Table 1

| Number of bound Fab fragments of anti-mouse IgG (Fc) antibodies per one mouse IgG antibody molecule | RF-induced non-specific agglutination reactions[1] (RF:653 IU/ml) | | | | |
|---|---|---|---|---|---|
| | 1 min. | 2 min. | 3 min. | 5 min. | 10 min. |
| 0 | + + | + + | + + | + + | + + |
| 1.6 | - | ± | ± | ± | ± |
| 2.7 | - | - | - | - | ± |
| 3.1 | - | - | - | - | - |

[1] + + represents agglutination, and - represents non-agglutination.
The case where agglutination was absent with a slight variation is marked ±.

With the latex reagent that was prepared from the mouse IgG antibodies whose Fc portions were blocked with the Fab fragments of the anti mouse IgG (Fc) antibodies, no non-specific agglutination reaction caused by RF was observed.

Example 4

(1) Preparation of anti-myoglobin antibody-sensitized latex reagent

In 9.5 ml of glycine buffer was dissolved 1.5 mg of rabbit IgG-anti-myoglobin antibody, and 0.5 ml of 0.46 $\mu$m polystylene latex (suspension containing 10 weight % of solids) was added. After the mixture was stirred at room temperature for 2 hours, the sensitized latex was centrifuged, to remove the supernatant. The precipitate was suspended in 10 ml of glycine buffer containing 1.5 mg of Fab' fragment obtained according to the procedure described in Example 2, to block the Fc portions of the rabbit IgG antibodies. The suspension was further centrifuged to remove the supernatant. The precipitate was suspended in bovine serum albumin solution (0.1 M glycine, 0.05 M sodium chloride, 0.05% sodium azide and 0.2% BSA), to produce the anti-myoglobin antibody-sensitized latex reagent.

Further, the amount of IgG antibodies adsorbed to latex and the amount of Fab' fragments bound to those IgG antibodies were determined as described in Example 3, the molecular ratio was calculated, and the result was taken as the number (average) of the bound Fab' fragments of the anti rabbit IgG (Fc) antibodies per one rabbit IgG antibody molecule.

(2) Non-specific reaction with RF

A drop of an RF positive human serum (653 IU/ml; International Immunology Corporation) and a drop of the anti-myoglobin antibody-sensitized latex reagent prepared as described above were mixed, and the reaction plate was rolled gently, to observe the agglutination image for 10 min. The results obtained in the above described way were compared with results obtained with anti-myoglobin antibody-sensitized latex reagents differing in the number of the Fab' fragments (Example 2) used to block the Fc portions of the rabbit IgG antibodies as listed in Table 2.

Table 2

| Number of bound Fab fragments of anti-rabbit IgG (Fc) antibodies per one rabbit IgG antibody molecule | RF-induced non-specific agglutination reactions[1] (RF:653 IU/ml) | | | | |
|---|---|---|---|---|---|
| | 1 min. | 2 min. | 3 min. | 5 min. | 10 min. |
| 0 | + + | + + | + + | + + | + + |
| 1 | - | - | - | - | - |
| 2 | - | - | - | - | - |

[1] + + represents agglutination, and - represents non-agglutination.

With the latex reagent that was prepared from the rabbit IgG antibodies whose Fc portions were blocked with the Fab' fragments of the anti rabbit IgG (Fc) antibodies, no non-specific agglutination reaction caused by RF was observed.

(3) Determination of myoglobin content in serum

After one drop of serum from a subject and one drop of the latex reagent prepared in (1) were mixed, the reaction plate was rolled gently, while the agglutination image was observed. The agglutination image thus obtained, and an agglutination image obtained by the same method except for using the sensitized latex reagent that has left the Fc portions of the anti myoglobin IgG antibodies unblocked as a control, and an agglutination image obtained after a pretreatment that consisted of adding degenerate $\gamma$-globulin to the serum from the subject to a concentration of 2 mg/ml and of allowing the mixture to stand for 1 hour, were compared, and the results therefrom are shown in Table 3, together with the quantified data of RF obtained by using RF-latex "Seiken".

Table 3

| Sample No. | Agglutination reaction[1] | | | RF[2] (IU/ml) |
|---|---|---|---|---|
| | Method of the present invention | Reference methods | | |
| | | without pretreatment | with pretreatment | |
| 1 | − | − | − | 5.7 |
| 2 | − | − | − | 5.0 |
| 3 | − | +++ | − | 61.4 |
| 4 | − | +++ | − | 98.5 |
| 5 | + | + | + | 5.0 |
| 6 | + | + | + | 5.0 |
| 7 | ++ | ++ | ++ | 5.0 |
| 8 | ++ | +++ | ++ | 48.1 |
| 9 | ++ | +++ | ++ | 88.5 |
| 10 | +++ | +++ | +++ | 5.0 |

[1] +++ represents agglutination within 1 min., and ++ represents agglutination within 3 min. Further, + represents agglutination at 5 min. and = represents non-agglutination at 5 min.

[2] Determined by using COBAS[(R)] MIRA RF-latex "Seiken".

According to the control method (Fc portion left unblocked), in the samples #3 and #4, non-specific reactions due to RF were observed. Further, the samples #8 and #9 also seems to suffer non-specific agglutination reactions due to RF. On the other hand, in the measurement wherein degenerate $\gamma$-globulin was added to the sample as a pretreatment, no non-specific agglutination reaction due to RF was observed, and in the measurement with the reagent of the present invention, no non-specific agglutination reaction was observed, either.

Example 5

(1) Preparation of Fab blocking agent by papain digestion of goat IgG-anti-mouse IgG (Fc) antibody

After goat IgG-anti IgG (Fc) antibodies had been digested with papain as in Example 1, 10 mM iodoaceto amide was added to the mixture in a dark place, and incubated at 37 °C for 15 min. The reaction solution was dialyzed against 100 mM glycine buffer (pH 8.6), to obtain an admixture comprising Fab fragments (blocking agents) and Fc fragments.

EP 0 566 205 A1

**(2) Preparation of anti-D-Dimer antibody-sensitized latex reagent (reagent A) (control method)**

In 9.5 ml of sodium phosphate buffer was dissolved 1.25 mg of mouse IgG-anti D-Dimer antibody, to which was added 0.5 ml of 0.65 μm polystylene latex (buffer containing 10 weight % solids), and the mixture was stirred at room temperature for 2 hours. Then, the sensitized latex reagent was centrifuged, to discard the supernatant. The precipitate was suspended in bovine albumin solution (0.1 M glycine, 0.05 M sodium chloride, 0.05% sodium azide and 0.2% BSA), to produce the anti-D-Dimer antibody-sensitized latex reagent.

**(3) Blocking with unpurified Fab fragments prepared in (1), of Fc portion of mouse IgG-anti-D-Dimer antibody adsorbed to latex in the reagent A**

To 0.5 ml of the reagent A was added 2.25 mg of the unpurified Fab fragments prepared in (1), and the mixture was incubated at 37 °C for 30 min., and then was centrifuged to remove the supernatant. The precipitate was suspended in bovine serum albumin solution, to produce the reagent B.

**(4) Blocking with purified Fab fragments obtained in Preparation Example 1, of Fc portion of mouse IgG-anti-D-Dimer antibody adsorbed to latex in the reagent A**

To 0.5 ml of the reagent A was added 300 μg of the purified Fab fragments obtained in Example 1, and the mixture was incubated at 37 °C for 30 min., and then was centrifuged to remove the supernatant. The precipitate was suspended in bovine serum albumin solution, to produce the reagent C.

**(5) Non-specific reactions with RFs**

Responses obtained with RF positive control 100 IU/ml (Japan Roche), and the reagents A, B and C prepared as above were compared. A drop of the RF positive control was added to one drop of each of the reagents A, B and C, and the solution was mixed. Then, the reaction plate was rolled gently, while agglutination images were observed for 5 minutes. The results are given in Table 4.

Table 4

| Reagent | RF-induced non-specific agglutination reactions | | | | RF.Mb negative serum 5 min. |
| | Reaction time | | | | |
| | 1 min. | 2 min. | 3 min. | 5 min. | |
| A (control) | + | + | ++ | ++ | - |
| B | - | - | - | - | - |
| C | - | - | - | - | - |

1) ++ represents agglutination, and
- represents non-agglutination,
+ represents weak agglutination.

With the reagent A (control), non-specific reaction due to RF was observed, whereas with the reagents B and C that had been treated by adding the blocking agent (Fab fragment of anti mouse IgG (Fc) antibody) to the reagent A, no non-specific agglutination reaction was observed.

9

Example 6

(1) Blocking of Fc fragment of solid antibody in EIA sandwich process:

100 μl of the blocking reagent obtained in Example 1 was added to each well of micro plate which was previously coated with mouse monoclonal antibody, anti-FgDP/anti-FbDP antibody. After incubation for 30 min. at an ambient temperature with slightly shaking, the liquid in each well was subjected to suction and a phosphate buffered solution was added to wash each well. This washing step was repeated three times to obtain wells in which the Fc fragment of the antibody was blocked. These wells thus obtained are hereinafter referred to as group B.

The molecular number of Fab fragment of anti mouse IgG(Fc) antibody of which Fc fragment was blocked in a manner as stated in the above was estimated by means of Protein Assay Kit (supplied by Bio Rad Lab. Inc.), and it was revealed that the Fc fragment of the solid antibody (mouse IgG) in B Group was bound by approximately one molecule of anti mouse IgG(Fc) antibody.

In lieu of the blocking agent aforementioned, 100 μl of phosphate buffered solution was added and the remaining steps were repeated in the same manner as above to obtain a contrast well, hereinafter referred to as Group A.

(2) Non-specific reaction with RF:

A series of samples having RF concentrations of 1421 IU/ml, 710 IU/ml, 89 IU/ml and 0 IU/ml were prepared by using 1421 IU/ml of human RF positive serum (supplied by International Immunology Corporation) and a phosphate buffered solution.

100 μl of each sample was added to both Group A and Group B, respectively, and an incubation was carried out for 15 min. at a temperature of 37 °C.

Each well was washed four times and 100 μl of mouse monoclonal antibody labelled with peroxidase was added (anti-FbDP antibody-HRP) followed by incubation for 15 min. at 37 °C.

Subsequently the well was further washed four times and 100 μl of a tetramethylbenzidine solution was added followed by incubation for 10 min. at 37 °C.

Then to each well was added 100 μl of an aqueous 1 M sulphonic acid solution to terminate the enzymatic reaction.

After the termination of the reaction, an optical absorption at 450 nm was determined by means of a microtiter plate reader (Reader 510 manufactured by Organon Teknika KK). The results are tabulated in Table 5.

Table 5

| RF (IU/ml) | Absorption (450 nm) | |
|---|---|---|
| | Group A (Contrast) | Group B (Claimed Process) |
| 1421 | 0.820 | 0.320 |
| 710 | 0.511 | 0.225 |
| 89 | 0.098 | 0.063 |
| 0 | 0.037 | 0.039 |

As indicated in Table 5, it can be understood that Group B in which the blocking reagent was added to the solid antibody is effective in suppressing the non-specific reaction of RF in the EIA sandwich process in comparison to the contrast Group A to which no blocking agent was added.

**Claims**

1. An antibody for immunoassay, of which Fc portion is blocked by a blocking agent comprising at least an antigen-binding site of an immunoglobulin specifically bindable to the Fc portion.

2. An antibody according to claim 1, wherein said blocking agent comprises a fragment selected from the group consisting of Fv, Fab, Fab', F(ab')$_2$, Facb and Fab/c of said immunoglobulin.

3. An antibody according to claim 1, wherein said blocking agent is a fragment of the immunoglobulin and said fragment comprise one antigen-binding site.

4. An antibody according to claim 3, wherein said blocking agent is Fv, Fab or Fab'.

5. An antibody according to claim 4, wherein said Fc portion of the antibody is blocked by 0.5 to 6 Fab or Fab' fragments per portion on an average.

6. A reagent for immunoassay comprising the antibody as defined in any one of claims 1 to 5.

7. A reagent according to claim 6, wherein said antibody is adsorbed on a carrier.

8. A reagent according to claim 7, wherein said carrier is latex, glass or plastic.

9. A reagent according to claim 8, wherein said carrier is latex.

10. A reagent according to claim 6, wherein said antibody is adsorbed on a substrate.

11. A reagent according to claim 6, wherein said antibody is labeled.

12. A reagent according to claim 6, which is a latex reagent comprising a latex carrier sensitized with an IgG antibody, a Fc portion of said IgG antibody being blocked by Fab or Fab' fragment of a heterogeneous IgG antibody against said Fc portion.

13. An immunoassay using the reagent as defined in any one of claims 6 to 12.

14. An immunoassay according to claim 13, wherein said immunoassay is selected from single radial immunodiffusion (SRID), agglutination, radioimmunoassay (RIA), enzymeimmunoassay (EIA), turbidimetrical immunoassay (TIA), immunonephelometry, fluoroimmunoassay, luminesceimmunoassay, and turbidimetry, nephelometry and particle size distribution measurement based on agglutination technique.

15. A blocking agent for blocking Fc portion of an antibody for immunoassay, which comprises at least an antigen-binding site of an immunoglobulin specifically bindable to the Fc portion.

16. A blocking agent according to claim 15, which comprises a fragment selected from the group consisting of Fv, Fab, Fab', $F(ab')_2$, Facb and Fab/c of said immunoglobulin.

17. A blocking agent according to claim 15, which is a fragment of the immunoglobulin and said fragment has one antigen-binding site per fragment.

18. A blocking agent according to claim 17, which is Fv, Fab or Fab'.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | FR-A-2 365 800 (BEHRINGWERKE AK)<br>* page 1, line 29 - page 2, line 32 *<br>--- | 1-18 | C07K15/00<br>G01N33/531<br>G01N33/543<br>G01N33/546 |
| A | WO-A-8 807 089 (MEDICAL RESEARCH COUNCIL)<br>--- | | |
| A | EP-A-0 008 473 (AKZO N.V.)<br>--- | | |
| A | WO-A-9 118 291 (PB DIAGNOSTIC SYSTEMS, INC.)<br>--- | | |
| A | US-A-4 737 456 (L. WENG ET AL.)<br>----- | | |

| | TECHNICAL FIELDS SEARCHED (Int. Cl.5) |
|---|---|
| | G01N<br>C07K |

The present search report has been drawn up for all claims

| Place of search | Date of completion of the search | Examiner |
|---|---|---|
| THE HAGUE | 26 JULY 1993 | CARTAGENA ABELLA P |

EPO FORM 1503 03.82 (P0401)